# EUROPEAN PATENT SPECIFICATION

(11) **EP 4 563 209 B1**
(45) Date of publication and mention of the grant of the patent: **14.10.2026**
(21) Application number: 24852009.0
(22) Date of filing: 10.05.2024
(51) Int. Cl.: B01D 3/32, B01D 3/14, B01D 5/00, B01D 3/00, C07C 7/04, C07C 7/09, C07C 11/02, C07C 11/04, C07C 11/107

(54) **SEPARATION DEVICE**
TRENNVORRICHTUNG
DISPOSITIF DE SÉPARATION

(30) Priority: 08.08.2023 KR 20230103230
(43) Date of publication of application: 04.06.2025
(73) Proprietor: LG Chem, Ltd., Yeongdeungpo-gu Seoul 07336 (KR)
(72) Inventor: HWANG, Moon Sub, Daejeon 34122 (KR); LEE, Jeong Seok, Daejeon 34122 (KR); SONG, Jong Hun, Daejeon 34122 (KR); BAEK, Se Won, Daejeon 34122 (KR); PAK, Geun Tae, Daejeon 34122 (KR)
(74) Representative: Hoffmann Eitle
(86) International application number: PCT/KR2024/006342
(87) International publication number: WO 2025/033650

(56) References cited:
- EP-A1- 2 266 674
- CN-A- 110 002 960
- DE-A1- 10 002 806
- KR-B1- 101 805 875
- KR-B1- 102 362 742
- US-A1- 2023 002 685
- AGRAWAL R: "MULTICOMPONENT DISTILLATION COLUMNS WITH PARTITIONS AND MULTIPLE REBOILERS AND CONDENSERS", INDUSTRIAL AND ENGINEERING CHEMISTRY, PROCESS DESIGN ANDDEVELOPMENT., AMERICAN CHEMICAL SOCIETY. WASHINGTON., US, vol. 40, no. 20, 1 January 2001 (2001-01-01), US , pages 4256 - 4266, XP007901054
- CHEN ZEWEI, AGRAWAL RAKESH: "Classification and Comparison of Dividing Walls for Distillation Columns", PROCESSES, M D P I AG, CH, vol. 8, no. 6, CH , pages 699, XP093261421, ISSN: 2227-9717, DOI: 10.3390/pr8060699

## Description

This application claims priority to and the benefit of Korean Patent Application No. 10-2023-0103230 filed on August 08, 2023.

### [Technical Field]

The present disclosure relates to a separation device used in an oligomerization process using an ethylene monomer, and more particularly, to a separation device capable of preventing plugging problems due to polymer by-products and improving a recovery rate of unreacted ethylene by changing an inside of a sump of a distillation column to a double separation wall structure and improving a circulation system.

### [Background Art]

Alpha-olefin is widely used commercially as an important material used as a comonomer, a detergent, a lubricant, a plasticizer, or the like. In particular, 1-hexene and 1-octene has been widely used as a comonomer for controlling a density of polyethylene in a production of linear low-density polyethylene (LLDPE).

The alpha-olefin is typically prepared through the oligomerization reaction of ethylene, and a polymer is produced as a by-product during this preparation process. The polymer produced by a side reaction is contained in reactor effluents in an amount of about 1% by weight or less and is injected into the distillation column in a post-process. In this case, when the reactor emissions are directly injected into a feed stage tray, there is a very high risk of plugging phenomenon due to by-products such as polymers. Accordingly, a method of directly injecting reactor effluents into a reboiler section has been considered.

However, the reactor effluent contains an unreacted ethylene monomer in addition to by-products, and when the feed stage becomes the reboiler section, the separation efficiency of the unreacted ethylene monomer is greatly reduced. Accordingly, there is a problem that the entire amount of unreacted ethylene monomer may not be recovered from the reactor, and some of the unreacted ethylene monomer is leaked along with a bottom (BTM) product, and such loss of raw materials also causes financial loss.

Therefore, in order to solve the above problems, research is needed to prevent the plugging phenomenon due to polymer by-products in the separation process after the ethylene reaction in a post-process, and at the same time, minimize the loss of the unreacted ethylene monomer.

CN 110 002 960 A discloses a device for separating an (acetone-butanol-ethanol) ABE fermentation pervaporation liquid.

DE 10 002 806 A1 discloses a column separating a mixture of three or more substances.

### [Disclosure]

### [Technical Problem]

The problem to be solved by the present disclosure is to solve the problems mentioned in the Background Technology of the above disclosure, and an object of the present disclosure provides a separation device capable of preventing a plugging phenomenon due to polymer by-products in a post-process of an oligomerization reaction process, and at the same time, recovering an unreacted ethylene monomer without being lost to the lower portion of the distillation column by improving a sump structure at a lower portion of a distillation column and a circulation system.

However, problems to be solved by the present disclosure are not limited to the above-mentioned aspects. That is, other aspects that are not described may be obviously understood by those skilled in the art from the following description.

### [Technical Solution]

To solve the above problem, according to an embodiment of the present disclosure, a separation device includes a distillation column (2) having a double separation wall (10, 20), a first reboiler (3), a second reboiler (4), and a condenser (5). An inside of the distillation column (2) is partitioned into a bottom area (A), a middle area (B); and a top area (C).

The bottom area (A) of the distillation column (2)includes first to third areas (D1, D2, D3)that are partitioned by a first separation wall (10) and a second separation wall (20) extending from a bottom of the distillation column (2) to a predetermined height and spaced apart from each other, a raw material supply port (40) that is provided at one side of the first area (D1) and has a reactor effluent containing a monomer and an oligomer to the first area (D1) transferred therethrough, a first outlet (51) that is provided at a lower portion of the first area (D1) and connected to one side of the first reboiler (3) by a first flow path (110), a first reflux inlet (61) that is provided at an upper portion of the first area (D1) and connected to the other side of the first reboiler (3) by a second flow path (120), a second outlet (53) that is provided at a lower portion of the second area (D2) located between the first dividing wall (10) and the second dividing wall (20), and connected to one side of the second reboiler (4) by a third flow path (130), and a second reflux inlet (63) that is provided on one side of the third area (D3) and connected to the other side of the second reboiler (4) by a fourth flow path, wherein the first area (D1) and the second area (D2) are partitioned by the first separation wall (10) provided therebetween, and the second area (D2) and the third area (D3) are partitioned by the second separation wall (20) provided therebetween, wherein the distillation column (2) includes a plurality of trays (30) spaced apart in a vertical direction of the double separation wall (10, 20) in the middle region (B), and wherein the first reflux inlet (61) is located at a height between a lowermost tray among the plurality of trays (30) and the raw material supply port (40).

### [Advantageous Effects]

According to the separation device of the present disclosure, by improving the structure of the distillation column sump and the circulation system, it is possible to minimize the risk of plugging (fouling) of the internal structure of the column that may occur due to the polymer by-products contained in the reactor effluent and recover the entire amount of unreacted monomer dissolved in the solution to the reactor without being lost to the lower portion.

More specifically, by providing the double separation wall at the lower portion of the distillation column to divide the sump into a total of 3 areas and by using two reboilers with different flow rates, it is possible to function as a complete first stage of the column.

Effects which can be achieved by the present disclosure are not limited to the above-described effects. That is, other effects that are not described may be obviously understood by those skilled in the art to which the present disclosure pertains from the following description.

### [Description of Drawings]

FIG. 1 is a process diagram of a separation device according to an embodiment.
FIG. 2 is a diagram illustrating in detail a structure of a bottom area of a distillation column in the separation device according to the embodiment.
FIG. 3 is a diagram illustrating a conventional separation system as Comparative Example.

### [Mode for Invention]

Terms and words used in the present specification and claims are not to be construed as a general or dictionary meaning but are to be construed as meaning and concepts meeting the technical ideas of the present disclosure based on a principle that the inventors can appropriately define the concepts of terms in order to describe their own disclosures in best mode.

Throughout the accompanying drawings, similar or related components will be denoted by similar reference numerals.

A singular form of a noun corresponding to an item may include one or more of the items, unless the relevant context clearly dictates otherwise.

In the present disclosure, each phrase such as "A or B," "at least one of A and B," "at least one of A or B," "A, B, or C," "at least one of A, B and C," and "at least one of A, B, or C" may include any one of items listed together in the corresponding one of those phrases, or all possible combinations thereof.

The term "and/or" includes a combination of a plurality of related described components or any one of the pluralities of related described components.

Terms such as "first," "second," "1^{st}," or "2^{nd}" may simply be used to distinguish a component from another component, and do not limit the components in other respects (e.g., importance or order).

In addition, the terms such as 'front', 'rear', 'top', 'bottom', 'side', 'left', 'right', 'upper', 'lower', etc., used herein are defined based on the drawings, the shape and location of each component are not limited by this term.

It will be understood that terms 'include' or 'have' used in the present disclosure, specify the presence of features, numerals, steps, operations, components, parts mentioned in the present specification, or a combination thereof, but do not preclude the presence or addition of one or more other features, numerals, steps, operations, components, parts, or a combination thereof.

When a component is "connected," "coupled," "supported," or "contacted" with another component, this includes not only cases where the components are directly connected, coupled, supported, or contacted, but also cases where the components are indirectly connected, coupled, supported or contacted through a third component.

When a component is located "on" another component, this includes not only cases where a component is in contact with another component, but also cases where another component exists between the two components.

The terms "about," "substantially," or the like used throughout the present specification means figures corresponding to manufacturing and material tolerances specific to the stated meaning and figures close thereto, and are used to prevent unconscionable abusers from unfairly using the disclosure of figures precisely or absolutely described to aid the understanding of the present disclosure.

The term 'stream' used herein may mean a flow of a fluid in a process, and may also mean a fluid itself flowing in a pipe. Specifically, the stream may mean both the fluid itself and the flow of the fluid flowing within the pipe connecting each device. In addition, the fluid may include any at least one components of a gas, a liquid, and a solid.

Hereinafter, a separation device of the present disclosure will be described with reference to the attached drawings, but the attached drawings are exemplary, and the scope of the separation device is not limited thereto.

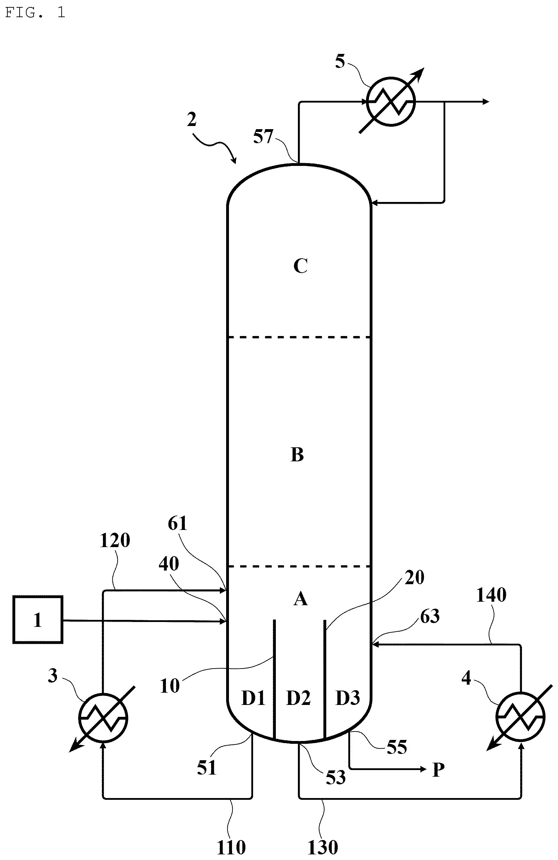

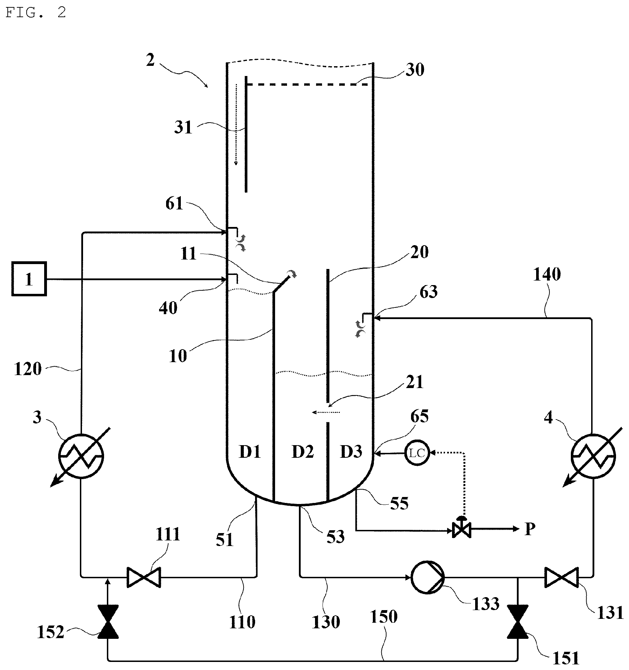

FIG. 1 is a diagram illustrating a separation device and a process flow according to an embodiment, and FIG. 2 is a diagram illustrating a detailed structure of a bottom area A of a distillation column according to an embodiment.

Referring to FIG. 1, the separation device according to an embodiment of the present disclosure includes a distillation column 2, a first reboiler 3, a second reboiler 4, and a condenser 5.

The distillation column 2 is a device that may separate multi-component substances contained in raw materials based on a difference in boiling points, respectively. Considering the boiling points of the inflowing raw material components, the components to be separated, a distillation column equipped with various types of trays 30 may be used in the separation device of the present disclosure. Furthermore, after an oligomerization process of an ethylene monomer, in order to minimize the risk of plugging due to by-products such as polymers while efficiently separating and recovering oligomer products and unreacted ethylene monomer contained in the reactor effluent discharged from the reactor 1, a distillation column equipped with double separation walls 10 and 20 at a bottom area A of the distillation column is used in the separation device of the present disclosure.

For example, as illustrated in FIG. 1, an inside of the distillation column 2 is partitioned into a bottom area A, a middle area B, and a top area C.

In this specification, the term "bottom area A" refers to a relatively lower portion in the structure of the distillation column 2, and refers to, for example, the lowermost portion of three areas partitioned in a height direction of the distillation column, and more specifically, an area under the lowermost tray, that is, the lower area.

In this specification, the term "top area C" refers to the relatively upper portion in the structure of the distillation column 2, and refers to, for example, an uppermost portion of three areas partitioned in the height direction of the distillation column, and more specifically, an area over the uppermost tray, that is, the upper area.

In addition, in this specification, "middle area B" refers to the relatively middle part in the structure of the distillation column 2, and refers to, for example, a middle area among three areas partitioned in the height direction of the distillation column, and more specifically, an area between the bottom area and top area of the distillation column, that is, an area between the lowermost tray and the uppermost tray.

In one embodiment, the bottom area A of the distillation column 2 is provided with a first separation wall 10, a second separation wall 20, a raw material supply port 40, a first outlet 51, a first reflux inlet 61, a second outlet 53, and a second reflux inlet 63, and may be further provided with a third outlet 55 and/or a third reflux inlet 65.

The separation device according to one embodiment may minimize the risk of plugging inside a column due to polymer by-products contained in the reactor effluent by providing the raw material supply port 40 in the bottom area A, and recover the entire amount of unreacted ethylene monomer to the reactor 1 without being lost to the bottom by changing a structure of a sump of the distillation column 2 using a double separation wall.

In one embodiment, the bottom area A of the distillation column 2 is provided with the double separation walls 10 and 20 in the sump where the liquid reactor effluent is collected. Here, the sump may refer to an area where a solution is collected at a lower portion of the distillation column. In addition, the bottom area A may be partitioned into a liquid area and a gas phase area based on a liquid surface of a solution.

More specifically, referring to FIG. 1, the bottom area A of the distillation column 2 has a first area D1, a second area D2, and a third area D3 partitioned by a first separation wall 10 and a second separation wall 20 that extend from the bottom of the distillation column 2 to a predetermined height and are spaced apart from each other. The first area D1 and the second area D2 are partitioned with the first separation wall 10 therebetween, and the second area D2 and the third area D3 are partitioned by a second separation wall 20 provided therebetween. In addition, the second area D2 is located between the first separation wall 10 and the second separation wall 20. Furthermore, it is preferable that the first area D1, the second area D2, and the third area D3 partitioned by the first separation wall 10 and the second separation wall 20 are partitioned equally.

Meanwhile, the height of the first separation wall 10 and the second separation wall 20 may be appropriately changed depending on the structure or size of the distillation column. However, as illustrated in FIG. 1, it may be provided so as not to contact the lowermost tray among the plurality of trays 30, which will be described later. Here, the predetermined height refers to a height at which the first separation wall 10 and the second separation wall 20 are spaced apart so as not contact the lowermost tray. For example, it may be preferable for the first separation wall 10 and the second separation wall 20 to have the same height, but the height is not limited thereto.

In addition, upper ends of the first separation wall 10 and the second separation wall 20 are provided so as not to contact the lowermost tray among the plurality of trays 30 to be described later, so gas in a gas phase area of the bottom area A may be able to move between the first area D1, the second area D2, and the third area D3.

Meanwhile, referring to FIG. 2, an auxiliary tray 11 having a predetermined slope in a direction of the second area D2 may be further provided at an upper end of the first separation wall 10. The auxiliary tray 11 is provided to compensate for the phenomenon in which the solution injected into the first area D1 does not pass through the first reboiler 3 but is bypassed to the second area D2. For example, the auxiliary tray 11 is inclined at an angle of less than 90°, more specifically about 40° to about 80°, in the direction of the second area D2 with respect to the first separation wall 10, but is not limited thereto. In addition, when the first separation wall 10 is provided with the auxiliary tray 11, the overall height including the auxiliary tray may be the same as the height of the second separation wall 20, but is not limited thereto.

In one embodiment, one area of the second separation wall 20 may be provided with an opening 21. Referring to FIG. 2, the opening 21 is located below the liquid surface of the solution in the second area D2 and the third area D3 to allow the solution to flow from the third area D3 to the second area D2, so the level of the solution inside two areas may be maintained the same. The shape and/or size of the opening 21 may be applied without particular limitation as long as it allows the solution to flow from the third area D3 to the second area D2. For example, the opening 21 may include one or more holes, and may also include a plurality of holes having different diameters. The shape, size, and/or number of the holes are not particularly limited as long as the level of the solution inside the second area D2 and the third area D3 may be maintained the same.

Referring to FIG. 1, the raw material supply port 40, the first outlet 51, and the first reflux inlet 61 are provided in the first area D1 of the separation device according to one embodiment.

In one embodiment, the raw material supply port 40 is connected to the reactor 1 and provided on one side of the first area D1, so the reactor effluent containing the ethylene monomer and oligomer discharged from the reactor 1 may be supplied to the first area D1. More specifically, the raw material supply port 40 is located on the side of the first area D1 of the distillation column 2 to have a height adjacent to the height of the first separation wall 10, and is preferably provided at a height higher than the liquid surface of the solution in the first area D1.

Here, the reactor effluent includes an oligomer product (alpha-olefin, P) produced by the oligomerization reaction of the ethylene monomer performed in the reactor 1 and the ethylene as the unreacted monomer, and may further include a polymer as a by-product. As in the related art, when the reactor effluent is injected into the feed stage tray located in the middle area of the column, there is the risk of plugging phenomenon due to the polymer by-product. However, in the present disclosure, the raw material supply port 40 is located in the bottom area A to prevent the column plugging phenomenon.

The oligomerization reaction may refer to a reaction in which a monomer is polymerized. Depending on the number of monomers to be polymerized, trimerization and tetramerization are called, and these trimerization and tetramerization are collectively called multimerization. Alpha-olefin is widely used commercially as an important material used as, e.g., a comonomer, a detergent, a lubricant, or a plasticizer. In particular, 1-hexene and 1-octene has been widely used as a comonomer for controlling a density of polyethylene in a production of linear low-density polyethylene (LLDPE). The alpha-olefins such as 1-hexene and 1-octene may be produced, for example, through trimerization or tetramerization of ethylene.

Meanwhile, in one embodiment, the first outlet 51 is provided at the bottom of the lower portion of the first area D1 and connected to one side of the first reboiler 3 by a first flow path 110. A lower discharge stream of the first area D1 containing the reactor effluent, that is, the first stream, may be transferred to the first reboiler 3 through the first outlet 51 to perform primary heat exchange. Since both the reactor effluent containing the unreacted monomers and the dropping liquid from the lowermost tray flow into the first area D1, a flow rate of about 80 vol% or more, and preferably about 90 vol% or more, may be discharged through the first outlet 51 located at the lower portion of the first area D1 and induced to pass through the first reboiler 3.

In addition, as illustrated in FIG. 2, the first flow path 110 may be further provided with a valve 111 that can be opened and closed as needed.

In the present application, the reboilers 3 and 4 are heating devices separately installed outside the distillation column 2, and may refer to a device for reheating and evaporating the flow of high boiling point components discharged from the bottom of the distillation column.

In one embodiment, the first reboiler 3 may be a device that heats the first stream discharged from the lower portion of the first area D1 of the distillation column 2 through the first flow path 110 by primary heat exchange. For example, it may be preferable to use a thermosyphon reboiler as the first reboiler 3. The thermosyphon reboiler is operated by a method of continuously supplying liquid at a lower portion of a distillation column to a reboiler with no power without a pump, and may be designed in which heat is supplied in the form of two phases of gas and liquid due to gas evaporating from the reboiler and a density is greatly reduced compared to the liquid at an inlet due to the gas, and thus, the natural circulation of the liquid continues to be performed stably due to the density difference and the liquid head pressure inside the distillation column. There may be a certain percentage of polymer by-products in the sump of the distillation column, but it is determined that the amount of the polymer by-products is not only extremely small, but the polymer by-products are in a liquefied state under high temperature conditions. Therefore, by performing the primary heat exchange through the thermosyphon recirculation system that can be installed and operated at low cost, there is an advantage in that a large amount of circulation flow can be operated economically and efficiently without additional devices such as a pump.

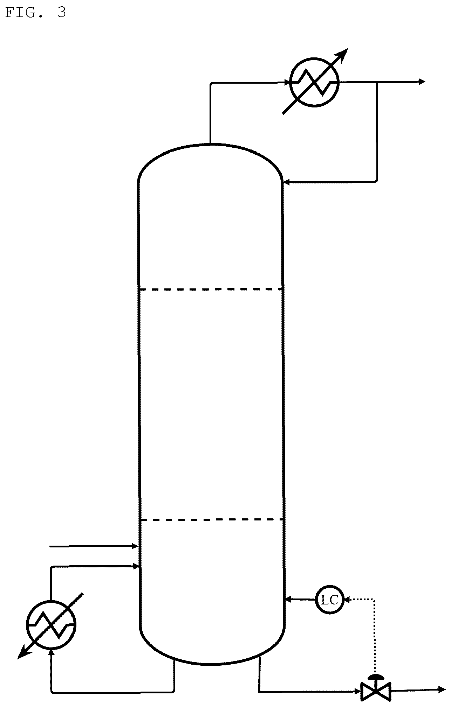

Meanwhile, FIG. 3 illustrates a conventional separation device without a separation wall in the distillation column sump as a comparative example. As illustrated in FIG. 4, when the reactor effluent is injected into the bottom area where the separation wall is not provided, the areas of the liquid stream discharged to the reboiler and the liquid stream where the product is discharged are not separated. Accordingly, there is a problem in that the entire amount of reactor effluent does not pass through the reboiler, and the unreacted monomers along with the products may be discharged directly to the bottom.

On the other hand, as described above, in the separation device according to an embodiment of the present disclosure, all the solution discharged from the reactor 1, a two-phase fluid that passes through the first reboiler 3, and the liquid separated and dropped after heat exchange in the column lowermost tray 30 flows into the first area D1 and overflow by at a flow rate that exceeds the amount discharged to the lower portion of the first area D1 and pass into the second area D2. Here, the lower discharge stream (first stream) of the first area D1 performs the primary heat exchange through the thermosyphon recirculation system of the first reboiler 3. In this case, about 80 vol% or more and preferably 90 vol% or more of the total flow rate may be circulated to the first reboiler 3. As a result, there is the advantage of increasing the recovery rate of the unreacted ethylene monomer.

In addition, the lower discharge stream (first stream) of the first area D1 may be heated to a temperature of about 200°C to 205°C by the primary heat exchange in the first reboiler 3. By heating the first stream to the above temperature range through the primary heat exchange, it is possible to prevent the pipe plugging phenomenon due to the polymer by-products while converting the unreacted ethylene monomer dissolved in the solution into the vapor phase.

In one embodiment, the first reflux inlet 61 is located in the upper portion of the first area D1, and may be connected to the other side of the first reboiler 3 by the second flow path 120 and provided on the side portion of the distillation column 2. The first stream that has passed through the first reboiler 3 through the first reflux inlet 61 may be refluxed back to the first area D1.

More specifically, the first reflux inlet 61 is located at a height between the lowermost tray of the plurality of trays 30, which will be described later, and the raw material supply port 40. The reactor effluent flowing from the reactor 1 through the raw material supply port 40, the first stream discharged to the first outlet 51, passed through the first reboiler 3, and then recycled through the first reflux inlet 61, and/or the liquid separated and dropped from the lowermost tray 30, which will be described later, flow into the first area D1. In this way, when the flow rate flowing into the first area D1 exceeds the capacity that may be accommodated in the first area D1, the solution in the first area D1 passes into the first separation wall 10 and is moved to the second area D2. In this case, since the first reflux inlet 61 is located at a higher height than the raw material supply port 40, the solution of the first heat-exchanged first stream refluxed through the first reflux inlet 61 preferentially passes into the second area D2.

Referring to FIG. 1, according to one embodiment, the second outlet 53 is provided in the second area D2 and the second reflux inlet 63 is provided in the third area D3. In addition, as illustrated in FIG. 2, the third outlet 55 and/or the third reflux inlet 65 may be additionally provided in the third area D3.

In one embodiment, the second outlet 53 may be provided at the bottom of the lower portion of the second area D2 located between the first separation wall 10 and the second separation wall 20, and connected to one side of the second reboiler 4 by the third flow path 130. The secondary heat exchange may be performed by transferring the lower discharge stream of the second area D2, that is, the second stream, to the second reboiler 4 through the second outlet 53. In addition, as illustrated in FIG. 2, the third flow path 130 may be further provided with the valve 131 that can be opened and closed and/or the pump 133 as needed.

The second reboiler 4 may be a device that heats the second stream flowing out through the third flow path 130 from the lower portion of the second area D2 of the distillation column 2. For example, the second reboiler may be preferably a forced circulation reboiler. The forced circulation reboiler uses the pump 133 to forcibly supply liquid to the reboiler, and may be used for when the forced circulation is required, such as a fluid with high viscosity or when the fluid is sensitive to temperature and requires rapid reboiler circulation. When the forced circulation reboiler is used in the reboiler circulation in the second area D2, in the case where the efficiency of the thermosyphon reboiler in the first area D1 decreases over time due to the influence of high viscosity of the polymer solution, etc., as the flow rate of the first stream discharged to the first outlet 51 decreases, the heat exchange capacity in the first reboiler 3 decreases, so the reflux flow rate of the entire distillation column may also decrease. In this case, by adjusting the relative ratio of the circulation flow rates between the first reboiler 3 and the second reboiler 4, the second reboiler 4 compensates for the decrease in efficiency of the first reboiler 3, so the second boiler 4 can be operated for a long period of time.

In addition, the lower discharge stream (second stream) of the second area D2 may be heated to a temperature of about 205°C to 210°C by the secondary heat exchange in the second reboiler 4. By heating to the above temperature range, the unreacted ethylene monomer remaining in the solution may be converted into the vapor phase and the plugging phenomenon due to the polymer by-products may be prevented.

In one embodiment, the second reflux inlet 63 is provided in one side of the third area D3, and is connected to the other side of the second reboiler 4 by the fourth flow path 140 and provided on the side portion of the distillation column 2. The second stream that has passed through the second reboiler 4 through the second reflux inlet 63 may be refluxed to the third area D3. More specifically, the second reflux inlet 63 may be located at a height between the upper end of the second separation wall 20 and the liquid surface of the solution in the third area D3.

As described above, the liquid flowing into the second area D2 in excess of the capacity that may be accommodated in the first area D1 may flow beyond the first separation wall 10. Furthermore, the second stream is discharged to the second outlet 53 at the lower portion of the second area D2, passes through the second reboiler 4, and then recirculated through the second reflux inlet 63 may flow into the third area D3, and some of the solution in the third area D3 may flow back into the second area D2 through the opening 21 provided in the second separation wall 20. As a result, the solution level inside the second area D2 and the third area D3 may be maintained the same. Furthermore, the average temperature of the solution in the second area D2 may increase due to the solution flowing from the third area D3, so the third flow path 130 through which the second stream, which is the lower discharge stream of the second area D2, passes, may prevent the problem of the pipe plugging due to the polymer by-products.

In addition, the bottom area A of the distillation column 2 is partitioned into a lower stream area of the first area D1 that discharges the reactor effluent to the first reboiler 3, a lower stream area of the second area D2 that discharges the solution overflowing from the primary heat-exchanged liquid stream passing through the first reboiler 3 to the second reboiler 4, and a lower stream area of third area D3 discharging a product P by the first separation wall 10 and the second separation wall 20, and the raw material supply port 40 is located in the first area D1, so the flow rate of about 80 vol% or more of the reactor effluent, preferably about 90 vol% of the reactor effluent is designed to pass through the first reboiler 3, thereby further improving the separation efficiency of the unreacted monomers.

However, in the reaction device according to the present disclosure, in order to prevent the plugging of the structure in the column due to the polymer by-products contained in the reactor effluent, the reactor effluent feed is directly injected into the sump of the distillation column, so some of the unreacted ethylene monomer dissolved in the solution is still included in the solution and may not be separated into vapor and may pass into the second area D2. To compensate for this, the lower discharge stream (second stream) of the second area D2 also performs secondary heat exchange through the forced circulation system of the second reboiler 4 to achieve the role of the first stage of a complete distillation column that separates unreacted ethylene monomer into a gas phase. Accordingly, ultimately, the entire amount of ethylene monomer, which is the unreacted reaction raw material, may be recovered to the upper portion of the distillation column, that is, to the top, without the plugging (fouling) of the structure in the column, making it possible to operate a stable and economical separation process.

Meanwhile, the flow rate ratio of the first stream flowing out through the first flow path 110 and the second stream flowing out through the third flow path 130 may be 7:3 to 9:1, and is preferably about 7.5:2.5 to 8.5:1.5. In this case, by satisfying the flow rate ratio, the sum of the heat amount of the first stream that flows out through the first flow path 110 and passes through the first reboiler 3 and the second stream that flows out through the third flow path 130 and passes through the second reboiler 4 may be maintained the same. Since the amount of heat required throughout the separation process is fixed, even if two reboilers are used as in the present disclosure, the total heat amount may be the same as the conventional separation process using one reboiler. More specifically, by using different types of reboilers with different flow rates together, it may function as the complete first stage of the column.

Meanwhile, the average temperature of the solution (i.e., liquid area) in the bottom area A of the distillation column 2 may range from about 195°C to 210°C, and preferably from about 200°C to 205°C. When the above temperature range is satisfied, appropriate heat exchange may be achieved by the first reboiler and the second reboiler, and an appropriate steam generation rate at the rear end of the reboiler may be maintained.

More specifically, the average temperature of the solution in the second area D2 may be relatively higher than that of the solution in the first area D1, and the average temperature of the solution in the third area D3 may be relatively higher than that of the solution in the second area D2. For example, the average temperature of the solution in the first area D1 of the distillation column 2 may range from about 195°C to 200°C, the average temperature of the solution in the second area D2 may range from about 200°C to 205°C, and the average temperature of the solution in the third area D3 may range from about 205°C to about 210°C. When each area satisfies the above temperature range, appropriate separation of vapor dissolved in the solution and efficient operation of two reboilers may be possible.

More specifically, the difference in the average temperature of the solution in the first area D1 and the solution in the second area D2 may be about 5°C to 10°C, and the difference in the average temperature of the solution in the second area D2 and the solution in the third area D3 may be about 2°C to 5°C. In particular, due to the difference in the average temperature between the solution in the second area D2 and the solution in the third area D3, the flowability of the solution contained in the relatively high-temperature third area D3 to the relative low-temperature second area D2 through the opening 21 may be improved. In addition, the effect of the temperature of the solution in the third area D3 being refluxed through the second reboiler 4 being transferred to the second area D2 through the opening 21 may be achieved. That is, the relatively high-temperature fluid present in the third area D3 moves to the second area D2 where the relatively low-temperature fluid exists and is mixed to increase the temperature of the liquid stream introduced into the second reboiler 4, thereby preventing the plugging of the pipe (i.e., the third flow path) by the polymer by-products in the stream.

Meanwhile, as illustrated in FIG. 2, the separation device according to one implementation may further include a preliminary flow path 150 connecting the first flow path 110 and the third flow path 130. Furthermore, the preliminary flow path 150 may be further provided with one or more valves 151 and 152 that can be opened and closed, and the first flow path 110 and/or the third flow path 130 may also be provided with one or more valves 111 and 131 that can be opened and closed, respectively.

For example, when the valve 131 of the third flow path is opened, the valves 151 and 152 of the preliminary flow path may be closed. In addition, when the valve 131 of the third flow path is closed, the valves 151 and 152 of the preliminary flow path may be opened. In addition, when the valves 151 and 152 of the preliminary flow path are opened, the valve 111 provided in the first flow path 110 may be closed. Furthermore, through the opening and closing operation of the valves 111, 131, 151, and 152 of each flow path, when necessary, the first reboiler 3 with a larger capacity may be converted into the forced circulation form by the pump 133 of the third flow path, and the second boiler 4 may also be used in a modified form that does not operate.

As described above, since the solution in the first area D1 has a relatively low average temperature, there is a possibility that the pipe plugging problem in the first flow path 110 may occur due to the polymer by-products in the first stream. In this way, when the efficiency of the thermosiphon reboiler, which is the first reboiler 3, decreases due to the accumulation of the polymer by-products, the circulation flow rate increases through the mechanical stroke control of the forced circulation pump 133 and the decrease in efficiency of the first reboiler 3 is compensated by adding the supply of steam heat from the second reboiler 4, thereby overcoming the problem due to the decrease in efficiency of the first reboiler 3.

In one embodiment, the third outlet 55 is provided at the bottom of the lower portion of the third area D3 of the distillation column 2. The third stream containing the oligomer product P may be discharged through the third outlet 55. Referring to FIG. 2, the third reflux inlet 65 may be connected to the third outlet 55, located below the liquid surface of the solution in the third area D3, and provided on the side portion of the distillation column 2. More specifically, some of the liquid stream P containing the oligomer discharged to the third outlet 55 is recovered, and the remaining of the stream may be branched and refluxed back to the third area (D3) through the third reflux inlet 65.

Meanwhile, referring to FIG. 3 illustrating the conventional separation device using the distillation column without the separation wall in the sump, when the reactor effluent is injected into the bottom area without the separation wall, the areas of the stream discharged from the reboiler and the stream discharged from the product are not separated, so the entire reactor effluent does not pass through the reboiler (heat exchanger). As a result, there is a problem of reducing the separation efficiency due to the discharge of the unreacted ethylene monomer to the bottom along with the product.

In order to improve the conventional problem, according to the separation device according to the present disclosure, the bottom area A of the distillation column 2 is partitioned into the first area D1 in which the bottom area A of the distillation column 2 is provided with the first outlet 51 through which the reactor effluent is discharged to the first reboiler 3, the second area D2 provided with the second outlet 53 through which the solution overflowing from the first area D1 is discharged to the second reboiler 4, and the third area D3 provided with the third outlet 55 through which an oligomer product P with a relatively high boiling point is discharged by the first separation wall 10 and the second separation wall 20, and the raw material supply port 40 is located on the first area D1, so the entire reactor effluent is designed to necessarily pass through the first reboiler 3 and/or the second reboiler 4, thereby maximizing the separation efficiency of the unreacted monomers.

Referring to FIG. 1, the middle area B of the distillation column 2 includes the plurality of trays 30. The plurality of trays 30 are arranged in a vertical direction of the double separation walls 10 and 20 and are spaced apart from each other. The gaseous stream containing the unreacted monomer gas generated in the bottom area A passes through the plurality of trays 30 provided in the middle area B, is separated into gas and liquid, and moves to the top area C which will be described later.

The number and type of the plurality of trays 30 may be appropriately selected in consideration of the type of distillation column 2 and the desired separation efficiency, and the type of tray may be used without particularly limited as long as it is a tray normally used in a distillation column for gas-liquid separation.

For example, the distillation column 2 may be a distillation column with 10 to 30 theoretical stages. In the above, "theoretical stages" refers to the number of stages partitioned by a virtual area or a plurality of trays in the distillation column 2 where two phases, such as gas phase and liquid phase, are in equilibrium with each other.

In the separation device according to an embodiment of the present disclosure, one end of the lowermost tray 30 among the plurality of trays is located in the upper portion of the first area D1, and the separation device may further include a guide partition wall 31 extending downward from one end of the lowermost tray 30. One end of the lowermost tray 30 may be open so as not to contact the side portion of the distillation column 2 on the first area D1.

More specifically, the dropping liquid separated from the lowermost tray 30 after contact with gas and liquid may be guided to the first area D1 through the guide partition wall 31. Since the unreacted ethylene monomer may be dissolved even in the solution (dropping liquid) dropping from the tray, the guide partition wall 31 is provided at the end of the lowermost tray to ensure that the solution dropping from the tray flows only into the first area D1 to prevent the product from being lost directly to the third outlet 55, which is the product P outlet, without passing through the first reboiler 3 and/or the second reboiler 4, thereby maximizing the recovery rate of the unreacted monomers.

Referring to FIG. 1, a fourth outlet 57 connected to the condenser 5 is provided at the upper portion of the top area C of the distillation column 2.

The condenser 5 is a device that is separately installed outside the distillation column 2, and refers to a device for cooling the flow discharged from the top of the distillation column by contacting it with cooling water flowing from the outside. For example, the condenser 5 may be a device that condenses the gaseous stream discharged upward from the top area C of the distillation column 2 through the fourth outlet 57.

More specifically, the gaseous stream containing the unreacted ethylene monomer may pass through the plurality of trays 30 in the middle area B, reach the top area C of the distillation column 2, and then discharged to the condenser 5 through the fourth outlet 57 as an upper gaseous stream. The upper gaseous stream discharged from the condenser 5 may be condensed and return to the reactor 1. In addition, some of the condensed top stream may be partitioned and recycled to the distillation column 2, but is not limited thereto.

Hereinabove, exemplary embodiments of the separation device according to the present disclosure have been described and illustrated in the drawings. However, the above description and the illustration of the drawings illustrate only the core components for understanding the present disclosure. In addition to the processes and devices in the description and illustrated in the drawings, the processes and devices not separately described or illustrated may be appropriately applied and used to implement the separation device according to the present disclosure.

### [Description of Reference Signs]

| | | | |
|---|---|---|---|
| 1: | Reactor | 2: | Distillation column |
| 3, 4: | Reboiler | 5: | Condenser |
| A: | Bottom area | B: | Middle area |
| C: | Top area | D1, D2, D3: | First area to third area |
| 10: | First separation wall | | |
| 11: | Auxiliary tray | 20: | Second separation wall |
| 21: | Opening | 30: | Tray |
| 31: | Guide partition wall | | |
| 40: | Raw material supp ly port | | |
| 51, 53, 55, 57: | First outlet to fourth outlet | | |
| 61, 63, 65: | First reflux inlet to third reflux inlet | | |
| 110, 120, 130, 140, 150: | Flow path | | |
| 111, 131, 151,152: | Valve | | |
| 133: | Pump | | |

## Claims

1. A separation device, comprising:
a distillation column (2) having a double separation wall (10, 20), a first reboiler (3), a second reboiler (4), and a condenser (5),
wherein an inside of the distillation column (2) is partitioned into a bottom area (A), a middle area (B), and a top area (C), and
the bottom area (A) of the distillation column (2) includes:
first to third areas (D1, D2, D3) that are partitioned by a first separation wall (10) and a second separation wall (20) extending from a bottom of the distillation column (2) to a predetermined height and spaced apart from each other;
a raw material supply port (40) that is provided at one side of the first area (D1) and has a reactor effluent containing a monomer and an oligomer to the first area (D1) transferred therethrough;
a first outlet (51) that is provided at a lower portion of the first area (D1) and connected to one side of the first reboiler (3) by a first flow path (110);
a first reflux inlet (61) that is provided at an upper portion of the first area (D1) and connected to the other side of the first reboiler (3) by a second flow path (120);
a second outlet (53) that is provided at a lower portion of the second area (D2) located between the first dividing wall (10) and the second dividing wall (20), and connected to one side of the second reboiler (4) by a third flow path (130); and
a second reflux inlet (63) that is provided on one side of the third area (D3) and connected to the other side of the second reboiler (4) by a fourth flow path (140),
wherein the first area (D1) and the second area (D2) are partitioned by the first separation wall (10) provided therebetween, and the second area (D2) and the third area (D3) are partitioned by the second separation wall (20) provided therebetween,
wherein the distillation column (2) includes a plurality of trays (30) spaced apart in a vertical direction of the double separation wall (10, 20) in the middle region (B), and
wherein the first reflux inlet (61) is located at a height between a lowermost tray among the plurality of trays (30) and the raw material supply port (40).

2. The separation device of claim 1, wherein the second separation wall (20) includes an opening (21) and the opening (21) is located below a liquid surface of the solution in the second area (D2) and the third area (D3).

3. The separation device of claim 1, further comprising:
a third outlet (55) that is provided at a lower portion of the third area (D3) and has a liquid stream containing the oligomer discharged therethrough.

4. The separation device of claim 3, further comprising:
a third reflux inlet (65) through which some of the liquid stream discharged from the third outlet (55) is branched and returns to the third area (D3).

5. The separation device of claim 1, further comprising:
a preliminary flow path (150) that connects the first flow path (110) and the third flow path (130).

6. The separation device of claim 5, wherein the third flow path (130) and the preliminary flow path (150) are each provided with at least one valve that is opened and closed, respectively,
when a valve (131) of the third flow path (130) is opened, a valve (151, 152) of the preliminary flow path (150) is configured to be closed, and
when the valve (131) of the third flow path (130) is closed, the valve (151, 152) of the preliminary flow path (150) is configured to be opened.

7. The separation device of claim 1, wherein the first reboiler (3) is a thermosyphon reboiler, and
the second reboiler (4) is a forced circulation reboiler provided with a pump (133).

8. The separation device of claim 1, wherein the first flow path (110) and the third flow path (130) are configured such that a flow rate ratio of a first stream flowing out through the first flow path (110) and a second stream flowing out through the third flow path (130) is 7:3 to 9:1.

9. The separation device of claim 1, wherein the second area (D2) and the third area (D3) are configured such that a solution present in the second area (D2) has an average temperature relatively lower than an average temperature of a solution present in the third area (D3).

10. The separation device of claim 1, further comprising
a guide partition wall (31) extending downward from one end of a lowermost tray among a plurality of trays (30),
wherein one end of the lowermost tray among the plurality of trays (30) is opened so as not to contact a side portion of the distillation column (2) on the first area (D1).

11. The separation device of claim 1, wherein an auxiliary tray (11) having a predetermined slope in a direction of the second area (D2) is provided at an upper end of the first separation wall (10).

## Patentansprüche

1. Trennvorrichtung, umfassend
eine Destillationskolonne (2) mit einer doppelten Trennwand (10, 20), einem ersten Rückverdampfer (3), einem zweiten Rückverdampfer (4) und einem Kondensator (5),
wobei das Innere der Destillationskolonne (2) in einen unteren Bereich (A), einen mittleren Bereich (B) und einen oberen Bereich (C) unterteilt ist, und
der untere Bereich (A) der Destillationskolonne (2) enthält:
einen ersten bis dritten Bereich (D1, D2, D3), die durch eine erste Trennwand (10) und eine zweite Trennwand (20) unterteilt sind, die sich vom Boden der Destillationskolonne (2) bis zu einer vorbestimmten Höhe erstrecken und voneinander beabstandet sind;
eine Rohstoffzufuhröffnung (40), die an einer Seite des ersten Bereichs (D1) vorgesehen ist und durch die ein Reaktorabfluss, der ein Monomer und ein Oligomer enthält, in den ersten Bereich (D1) geleitet wird;
einen ersten Auslass (51), der an einem unteren Abschnitt des ersten Bereichs (D1) vorgesehen ist und über einen ersten Strömungsweg (110) mit einer Seite des ersten Rückverdampfers (3) verbunden ist;
einen ersten Rückfluss-Einlass (61), der in einem oberen Abschnitt des ersten Bereichs (D1) vorgesehen ist und über einen zweiten Strömungsweg (120) mit der anderen Seite des ersten Rückverdampfers (3) verbunden ist;
einen zweiten Auslass (53), der an einem unteren Abschnitt des zweiten Bereichs (D2) vorgesehen ist, der zwischen der ersten Trennwand (10) und der zweiten Trennwand (20) angeordnet ist, und der über einen dritten Strömungsweg (130) mit einer Seite des zweiten Rückverdampfers (4) verbunden ist; und
einen zweiten Rückfluss-Einlass (63), der an einer Seite des dritten Bereichs (D3) vorgesehen ist und über einen vierten Strömungsweg (140) mit der anderen Seite des zweiten Rückverdampfers (4) verbunden ist,
wobei der erste Bereich (D1) und der zweite Bereich (D2) durch die dazwischen angeordnete erste Trennwand (10) voneinander getrennt sind, und der zweite Bereich (D2) und der dritte Bereich (D3) durch die dazwischen angeordnete zweite Trennwand (20) voneinander getrennt sind,
wobei die Destillationskolonne (2) eine Vielzahl von Böden (30) enthält, die im mittleren Bereich (B) in vertikaler Richtung der doppelten Trennwand (10, 20) voneinander beabstandet sind, und
wobei sich der erste Rückfluss-Einlass (61) auf einer Höhe zwischen einem untersten Boden der Vielzahl von Böden (30) und der Rohstoffzufuhröffnung (40) befindet.

2. Trennvorrichtung gemäß Anspruch 1, wobei die zweite Trennwand (20) eine Öffnung (21) enthält und die Öffnung (21) unterhalb einer Flüssigkeitsoberfläche der Lösung in dem zweiten Bereich (D2) und in dem dritten Bereich (D3) angeordnet ist.

3. Trennvorrichtung gemäß Anspruch 1, ferner umfassend
einen dritten Auslass (55), der an einem unteren Abschnitt des dritten Bereichs (D3) vorgesehen ist und durch den ein Flüssigkeitsstrom abgegeben wird, der das Oligomer enthält.

4. Trennvorrichtung gemäß Anspruch 3, ferner umfassend
einen dritten Rückfluss-Einlass (65), durch den ein Teil des aus dem dritten Auslass (55) austretenden Flüssigkeitsstroms abgezweigt ist und in den dritten Bereich (D3) zurückgeführt wird.

5. Trennvorrichtung gemäß Anspruch 1, ferner umfassend
einen vorläufigen Strömungsweg (150), der den ersten Strömungsweg (110) und den dritten Strömungsweg (130) verbindet.

6. Trennvorrichtung gemäß Anspruch 5, wobei der dritte Strömungsweg (130) und der vorläufige Strömungsweg (150) jeweils mit mindestens einem Ventil versehen sind, das geöffnet bzw. geschlossen wird,
wobei wenn ein Ventil (131) des dritten Strömungswegs (130) geöffnet ist, ein Ventil (151, 152) des vorläufigen Strömungswegs (150) so konfiguriert ist, dass es geschlossen ist, und
wenn das Ventil (131) des dritten Strömungswegs (130) geschlossen ist, das Ventil (151, 152) des vorläufigen Strömungswegs (150) so konfiguriert ist, dass es geöffnet ist.

7. Trennvorrichtung gemäß Anspruch 1, wobei der erste Rückverdampfer (3) ein Thermosiphon-Rückverdampfer ist und der zweite Rückverdampfer (4) ein mit einer Pumpe (133) versehener Rückverdampfer mit Zwangsumwälzung ist.

8. Trennvorrichtung gemäß Anspruch 1, wobei der erste Strömungsweg (110) und der dritte Strömungsweg (130) so konfiguriert sind, dass das Durchflussverhältnis eines durch den ersten Strömungsweg (110) austretenden ersten Stroms zu einem durch den dritten Strömungsweg (130) austretenden zweiten Strom 7:3 bis 9:1 beträgt.

9. Trennvorrichtung gemäß Anspruch 1, wobei der zweite Bereich (D2) und der dritte Bereich (D3) so konfiguriert sind, dass eine in dem zweiten Bereich (D2) vorhandene Lösung eine mittlere Temperatur aufweist, die relativ niedriger ist als die mittlere Temperatur einer im dritten Bereich (D3) vorhandenen Lösung.

10. Trennvorrichtung gemäß Anspruch 1, ferner umfassend
eine Führungstrennwand (31), die sich von einem Ende eines untersten Bodens der Vielzahl von Böden (30) nach unten erstreckt,
wobei ein Ende des untersten Bodens der Vielzahl von Böden (30) offen ist, um keinen Kontakt mit einem Seitenabschnitt der Destillationskolonne (2) im ersten Bereich (D1) herzustellen.

11. Trennvorrichtung gemäß Anspruch 1, wobei an einem oberen Ende der ersten Trennwand (10) ein Hilfsboden (11) vorgesehen ist, der eine vorbestimmte Neigung in Richtung des zweiten Bereichs (D2) aufweist.

## Revendications

1. Dispositif de séparation, comprenant :
une colonne de distillation (2) présentant une double paroi de séparation (10, 20), un premier rebouilleur (3), un deuxième rebouilleur (4), et un condenseur (5),
dans lequel l'intérieur de la colonne de distillation (2) est cloisonné en une zone inférieure (A), une zone intermédiaire (B), et une zone supérieure (C), et
la zone inférieure (A) de la colonne de distillation (2) inclut :
des première à troisième zones (D1, D2, D3) qui sont cloisonnées par une première paroi de séparation (10) et une deuxième paroi de séparation (20) s'étendant depuis un fond de la colonne de distillation (2) jusqu'à une hauteur prédéterminée et étant espacées l'une de l'autre ;
un orifice d'alimentation en matière première (40) qui est prévu au niveau d'un côté de la première zone (D1) et à travers lequel un effluent de réacteur contenant un monomère et un oligomère est transféré vers la première zone (D1) ;
une première sortie (51) qui est prévue au niveau d'une partie inférieure de la première zone (D1) et raccordée à un côté du premier rebouilleur (3) par un premier canal d'écoulement (110) ;
une première entrée de reflux (61) qui est prévue au niveau d'une partie supérieure de la première zone (D1) et raccordée à l'autre côté du premier rebouilleur (3) par un deuxième canal d'écoulement (120) ;
une deuxième sortie (53) qui est prévue au niveau d'une partie inférieure de la deuxième zone (D2) située entre la première paroi de séparation (10) et la deuxième paroi de séparation (20), et raccordée à un côté du deuxième rebouilleur (4) par un troisième canal d'écoulement (130) ; et
une deuxième entrée de reflux (63) qui est prévue sur un côté de la troisième zone (D3) et raccordée à l'autre côté du deuxième rebouilleur (4) par un quatrième canal d'écoulement (140),
dans lequel la première zone (D1) et la deuxième zone (D2) sont cloisonnées par la première paroi de séparation (10) prévue entre elles, et la deuxième zone (D2) et la troisième zone (D3) sont cloisonnées par la deuxième paroi de séparation (20) prévue entre elles,
dans lequel la colonne de distillation (2) inclut une pluralité de plateaux (30) espacés dans une direction verticale de la double paroi de séparation (10, 20) dans la région intermédiaire (B), et
dans lequel la première entrée de reflux (61) est située à une hauteur entre le plateau le plus bas parmi la pluralité de plateaux (30) et l'orifice d'alimentation en matière première (40).

2. Dispositif de séparation selon la revendication 1, dans lequel la deuxième paroi de séparation (20) inclut une ouverture (21) et l'ouverture (21) est située sous une surface de liquide de la solution dans la deuxième zone (D2) et la troisième zone (D3).

3. Dispositif de séparation selon la revendication 1, comprenant en outre :
une troisième sortie (55) qui est prévue au niveau d'une partie inférieure de la troisième zone (D3) et à travers laquelle un flux liquide contenant l'oligomère est évacué.

4. Dispositif de séparation selon la revendication 3, comprenant en outre :
une troisième entrée de reflux (65) à travers laquelle une partie du flux liquide évacué depuis la troisième sortie (55) est dérivée et retourne vers la troisième zone (D3).

5. Dispositif de séparation selon la revendication 1, comprenant en outre :
un canal d'écoulement préliminaire (150) qui raccorde le premier canal d'écoulement (110) et le troisième canal d'écoulement (130).

6. Dispositif de séparation selon la revendication 5, dans lequel le troisième canal d'écoulement (130) et le canal d'écoulement préliminaire (150) sont chacun munis d'au moins une vanne qui est ouverte et fermée, respectivement,
lorsqu'une vanne (131) du troisième canal d'écoulement (130) est ouverte, une vanne (151, 152) du canal d'écoulement préliminaire (150) est configurée pour être fermée, et
lorsque la vanne (131) du troisième canal d'écoulement (130) est fermée, la vanne (151, 152) du canal d'écoulement préliminaire (150) est configurée pour être ouverte.

7. Dispositif de séparation selon la revendication 1, dans lequel le premier rebouilleur (3) est un rebouilleur thermosiphon, et
le deuxième rebouilleur (4) est un rebouilleur à circulation forcée muni d'une pompe (133).

8. Dispositif de séparation selon la revendication 1, dans lequel le premier canal d'écoulement (110) et le troisième canal d'écoulement (130) sont configurés de sorte qu'un rapport de débit d'un premier flux s'écoulant vers l'extérieur à travers le premier canal d'écoulement (110) et d'un deuxième flux s'écoulant vers l'extérieur à travers le troisième canal d'écoulement (130) est de 7:3 à 9:1.

9. Dispositif de séparation selon la revendication 1, dans lequel la deuxième zone (D2) et la troisième zone (D3) sont configurées de sorte qu'une solution présente dans la deuxième zone (D2) présente une température moyenne relativement inférieure à une température moyenne d'une solution présente dans la troisième zone (D3).

10. Dispositif de séparation selon la revendication 1, comprenant en outre
une cloison de guidage (31) s'étendant vers le bas depuis une extrémité du plateau le plus bas parmi une pluralité de plateaux (30),
dans lequel une extrémité du plateau le plus bas parmi la pluralité de plateaux (30) est ouverte de façon à ne pas entrer en contact avec une partie latérale de la colonne de distillation (2) sur la première zone (D1).

11. Dispositif de séparation selon la revendication 1, dans lequel un plateau auxiliaire (11) présentant une pente prédéterminée dans une direction de la deuxième zone (D2) est prévu au niveau d'une extrémité supérieure de la première paroi de séparation (10).
